# EUROPEAN PATENT APPLICATION

(11) **EP 1 486 568 A1**
(43) Date of publication of application: **15.12.2004**
(21) Application number: 04013889.3
(22) Date of filing: 14.06.2004
(51) Int. Cl.: C12P 7/62, C12R 1/465

(54) **Streptomyces sp. CJPV 975652 capable of converting compactin to pravastatin and method for producing pravastin using the same**

(30) Priority: 12.06.2003 KR 2003037857
(71) Applicant: CJ Corp., Seoul (KR)
(72) Inventor: Lee, Chan Kyu, Seoul (KR); Kim, Deog Yeor, Seongbuk-gu, Seoul (KR); Suh, Jung Woo, Yangcheon-gu, Seoul (KR); Chang, Jun Hwan, Seoul (KR)
(74) Representative: Gowshall, Jonathan Vallance

(57) **Abstract**

Provided is a *Streptomyces* sp. CJPV 975652 (KCCM-10497) capable of converting compactin to pravastatin and a method for producing pravastatin using a strain of *Streptomyces* sp. CJPV 975652 (KCCM-10497), which includes: culturing the strain of *Streptomyces* sp. CJPV 975652 (KCCM-10497) in a compactin-containing medium; and recovering pravastatin from the culture.

## Description

### BACKGROUND OF THE INVENTION

This application claims priority from Korean Patent Application No. 2003-37857, filed on June 12, 2003, in the Korean Intellectual Property Office, the disclosure of which is incorporated herein in its entirety by reference.

### 1. Field of the Invention

The present invention relates to a novel *Streptomyces sp.* capable of converting compactin to pravastatin and a method for producing pravastatin using the same.

### 2. Description of the Related Art

Pravastatin and compactin are substances that are effective in the treatment of hyperlipidemia. Hyperlipidemia is a major risk factor for atherosclerosis and coronary artery syndrome which are major causes of death by cardiovascular diseases in advanced countries. Hyperlipidemia refers to an excess elevation of lipids, cholesterol, and neutral fats in the bloodstream due to disorder of lipoprotein metabolism. More than 60% of cholesterol in the body is derived from biosynthesis. More than 25 enzymes are involved in cholesterol biosynthesis. Among these enzymes, 3-hydroxy-3-methylglutaryl (HMG)-CoA reductase is a major rate-limiting enzyme in cholesterol biosynthesis. Therefore, selective inhibition of HMG-CoA reductase can effectively treat cholesterol associated diseases including hyperlipidemia. Compactin and pravastatin are competitive inhibitors of HMG-CoA reductase. When any one of compactin and pravastatin is present, cholesterol biosynthesis can be blocked. For example, it is known that daily dosage of 5-10 mg of pravastatin for 3 months reduces a total cholesterol level and a LDL-cholesterol (harmful in the body) level by 20-40%. Pravastatin may be prepared by hydroxylation of compactin by microorganism. Pravastatin is more effective than compactin as a HMG-CoA reductase inhibitor.

Among currently known antihyperlipidemic agents, simvastatin and atorvastatin are synthetic drugs that inhibit HMG-CoA reductase. Furthermore, lovastatin and pravastatin are representative drugs that can be produced by microorganism fermentation. Pravastatin is a drug that is biosynthesized by hydroxylation of compactin which is a precursor of pravastatin. Since pravastatin was first developed in early 1980's, efficacy and safety of pravastatin have been demonstrated. Therefore, pravastatin has been actively used in treating patients with hyperlipidemia.

It is reported that compactin can be converted to pravastatin by hydroxylation by microorganism including bacteria such as the genus *Nocardia* of the *Actinomycetes;* the genus *Actinomudura* of the *Maduromycetes;* and *Streptomyces roseochromogenus* and *Streptomyces carbophilus* of the *Streptomycetes,* and various genera of fungi (U.S. Patent Nos. 5,179,013, 4,448,979, 4,346,227, and 4,537,859, and Japanese Patent No. 58-10573).

However, a microorganism capable of producing pravastatin in high yield in a fermentation broth and an improved method for producing pravastatin using the microorganism are still being required.

### SUMMARY OF THE INVENTION

The present invention provides a novel *Streptomyces* strain capable of converting compactin to pravastatin in a high efficiency.

The present invention also provides a method for producing pravastatin, which includes converting comactin to pravastatin by the novel *Streptomyces* strain.

According to an aspect of the present invention, there is provided a *Streptomyces* sp. CJPV 975652 (KCCM-10497) capable of converting compactin to pravastatin.

According to another aspect of the present invention, there is provided a method for producing pravastatin using a strain of *Streptomyces* sp. CJPV 975652 (KCCM-10497), which includes: culturing the strain of *Streptomyces* sp. CJPV 975652 (KCCM-10497) in a compactin-containing medium; and recovering pravastatin from the culture.

### BRIEF DESCRIPTION OF THE DRAWINGS

The above and other features and advantages of the present invention will become more apparent by describing in detail exemplary embodiments thereof with reference to the attached drawings in which:
FIG. 1 is a HPLC chromatogram of pravastatin produced by *Streptomyces* sp. CJPV 975652 (KCCM-10497) of the present invention wherein the retention time of pravastatin is 12.766 minutes (area: 401298) and the retention time of compactin is 18.337 minutes (area: 230258);
FIG. 2 is a LC-MS spectrum of a HPLC fraction of pravastatin measured in negative mode wherein a peak of m/z 423 indicates pravastatin;
FIG. 3 is a MS daughter scan spectrum of a HPLC fraction of pravastatin measured in negative mode wherein a peak of m/z 423 indicates pravastatin and a peak of m/z 321 indicates a specific product obtained by fragmentation of pravastatin;
FIG. 4 is a UV spectrum of purified pravastatin;
FIG. 5 is a H-NMR spectrum of purified pravastatin; and
FIG. 6 is an electron microscopic image of *Streptomyces* sp. CJPV 975652 (KCCM-10497) of the present invention.

### DETAILED DESCRIPTION OF THE INVENTION

The present invention provides a strain of *Streptomyces* sp. CJPV 975652 (KCCM-10497) capable of converting compactin to pravastatin. The strain can convert compactin in a culture medium to pravastatin in high yield.

The present invention also provides a method for producing pravastatin using a strain of *Streptomyces* sp. CJPV 975652 (KCCM-10497), which includes: culturing the strain of *Streptomyces* sp. CJPV 975652 (KCCM-10497) in a compactin-containing medium and recovering pravastatin from the culture.

As used herein, the term "compactin" encompasses a lactone structure known as mevastatin, and its free acid and salt, as represented by the following Formulae 1, 2, and 3, respectively. In particular, the compound of Formula 3 is a sodium salt of compactin, although all pharmaceutically acceptable salts are intended to be encompassed by the present invention.

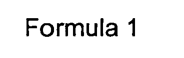

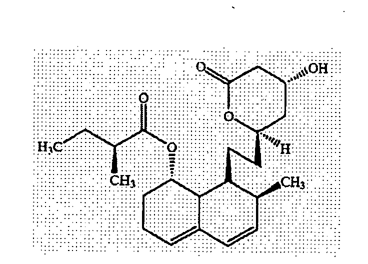

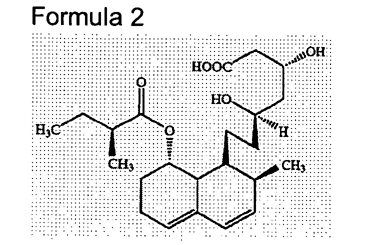

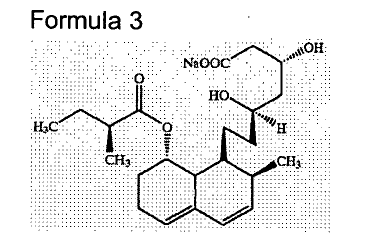

As used herein, the term "pravastatin" refers to a compound obtained by hydroxylation at the 6â-position of compactin and encompasses a lactone structure, and its acid and salt, as represented by the following Formulae 4, 5, and 6, respectively. The compound of Formula 6 is a sodium salt of pravastatin, although all pharmaceutically acceptable salts are intended to be encompassed by the present invention.

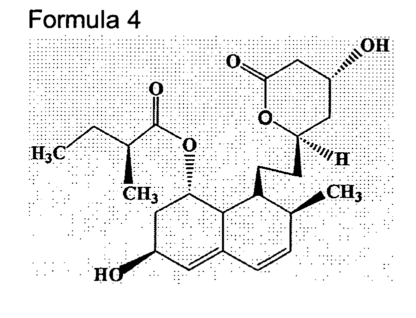

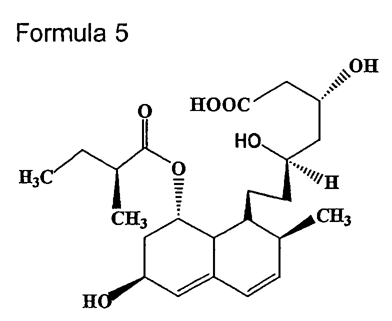

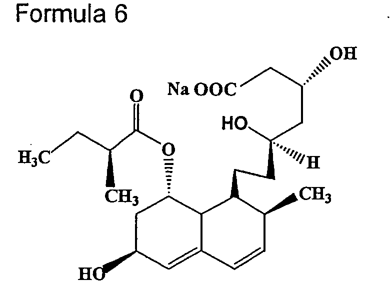

There are no particular limitations on the compactin-containing medium provided that it can be used in culturing common strains of the genus *Streptomyces.* The recovering pravastatin from the culture may be carried out using common separation and purification technique. For example, extraction and HPLC may be used.

Hereinafter, the present invention will be described more specifically by Examples. However, the following Examples are provided only for illustrations and thus the present invention is not limited to or by them.

### Example 1: Isolation of microorganism from soil

A soil sample was taken from a ginseng field (a district of Gangwha, Gyeonggi-do, Korea) and dried at 40°C for 24 hours. 1.0 g of the dried soil sample was suspended in 10ml of sterilized distilled water and consecutively twice diluted. 0.1 ml of the diluted solution was plated on 20ml of agar media (see Table 1) for separation of *Actinomyces* strains and cultured at 28°C for 14-21 days. Thereafter, soil microorganism was isolated. Humic acid-vitamin agar media containing 50 *µ*g/ml of cycloheximide as a fungicidal agent and 200 *µ*g/ml of nalidixic acid as an antibacterial agent were used as the agar media for separation of *Actinomyces* strains. The fungicidal agent and the antibacterial agent were separately filtration sterilized and then added to the humic acid-vitamin agar media that had been sterilized at 121°C for 15 minutes. The composition of the humic acid-vitamin agar media is summarized in Table 1 below.

**Table 1:**

| Composition of humic acid-vitamin agar media | | |
|---|---|---|
| Component | Content | Remark |
| Humic acid | 1.0g | 10 ml of 0.2N NaOH dissolution |
| Na₂HPO₄ | 0.5g | |
| KCI | 1.71 g | |
| MgSO₄ · 7H₂O | 0.05g | |
| FeSO₄ · 7H₂O | 0.01g | |
| Vitamin B group | 0.5mg | 0.5 mg of each of cyamin-HCl, B₂, niacin, pyridoxine-HCl, inositol, Ca-pentothetate, and p-amino benzoic acid, 0.25 mg of biotin |
| Cycloheximide | 50mg | |
| Nalidixic acid | 200*µ*g/ml | |
| Agar | 20g | |
| Distilled water | 1L | pH 7.0 |

*Actinomyces* colonies obtained according to the above method were purely separated. After compactin was added to *Actinomyces-*containing culture media, *Actinomyces* producing pravastatin from compactin were separated and designated as *Streptomyces* sp. CJPV 975652 (KCCM-10497).

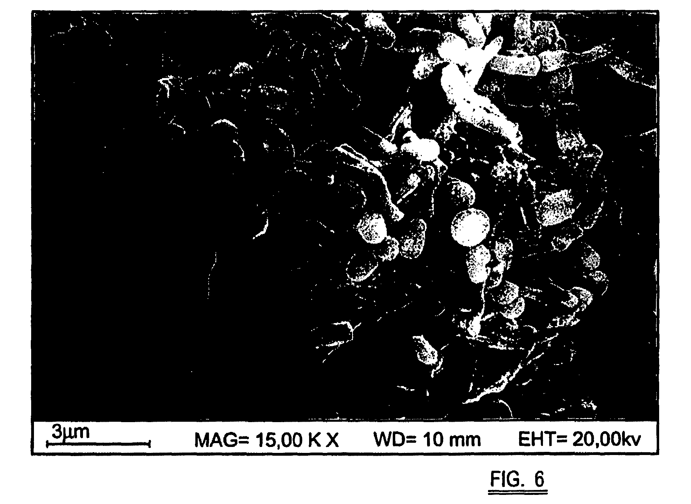

The growth of the microorganism was wholly good. Aerial mycelia exhibited mainly yellow or gray color with a pale yellow underneath. An electron microphotograph of the microorganism revealed straight spore chains, smooth spore surfaces, and fragmented spores (FIG. 6). The microorganism consumed most sugars contained in culture media, including D-glucose, sucrose, D-mannitol, D-fructose, D-xylose, L-arabinose, I-inositol, rhamnose, and raffinose. Gelatin liquefaction and starch digestion were positive. Furthermore, the sugar analysis of cell walls revealed glucose, arabinose, and galactose, and ribose, and the amino acid analysis revealed meso-diaminopimellic acid.

As a result of comparison between the separated microorganism and existing *Actinomyces* in terms of morphological, cultural, and physiological characteristics, the microorganism was judged to be a new strain of the genus *Streptomyces.* Therefore, the microorganism was designated as *Streptomyces* sp. CJPV 975652 and deposited in the Korean Culture Center of Microorganisms (KCCM), which is an international depository authority under the Budapest treaty, on May 12, 2003 (deposition number: KCCM-10497). The cultural characteristics of *Streptomyces* sp. CJPV 975652 (KCCM-10497) are summarized in Table 2 below.

**Table 2:**

| Cultural characteristics of *Streptomyces* sp. CJPV 975652 (KCCM-10497) | | | | |
|---|---|---|---|---|
| Medium | Growth | Color of aerial mycelia | Color of underneath | Water-soluble pigment |
| Tryptone-yeast extract agar medium | Good | Light yellow | Light brown | - |
| Yeast extract-malt extract agar medium | Excellent | Bright gray | Brown | - |
| Oatmeal agar medium | Good | White, pale yellow | Pale yellow | - |
| Inorganic salt-starch agar medium | Excellent | White, pale yellow | Yellow | - |
| Glycerol-asparagine agar medium | Good | Yellow, white | Light brown | - |
| Tyrosine agar medium | Excellent | Light yellow | Yellow, | Bright yellow |
| Nutrient agar medium | Normal | White, pale yellow | Yellow | - |
| Sugar agar medium | Excellent | Pale yellow | Yellow | - |
| Glucose-asparagine agar medium | Good | Pale yellow | Pale yellow | - |

Characteristics of the carbon source utilization of *Streptomyces* sp. CJPV 975652 (KCCM-1 0497) are summarized in Table 3 below.

**Table 3:**

| Characteristics of carbon source utilization of *Streptomyces* sp. CJPV 975652 (KCCM-10497) | |
|---|---|
| Carbon source | Utilization |
| D-glucose | + |
| L-arabinose | + |
| Sucrose | + |
| D-xylose | + |
| I-inositol | + |
| D-mannitol | + |
| D-fructose | + |
| Raffinose | + |

The physiological characteristics of *Streptomyces* sp. CJPV 975652 (KCCM-10497) are summarized in Table 4 below.

**Table 4:**

| Physiological characteristics of *Streptomyces* sp. CJPV 975652 (KCCM-10497) | |
|---|---|
| Section | Characteristics |
| Melanin production | Negative |
| Nitrate reduction | Positive |
| Starch hydrolysis | Positive |
| Gelatin liquefaction | Positive |
| Casein digestion | Negative |
| Casein coagulation | Positive |

The cell wall components of *Streptomyces* sp. CJPV 975652 (KCCM-10497) are summarized in Table 5 below.

**Table 5:**

| Cell wall components of *Streptomyces* sp. CJPV 975652 (KCCM-10497) | |
|---|---|
| Section | Component |
| DAP(diaminopimellic acid) | Meso-DAP |
| Glycine | - |
| Sugar | Sucrose |
| | Galactose |
| | Arabinose |
| | Ribose |

### Example 2: Identification of pravastatin produced from comactin by Streptomyces sp. CJPV 975652 (KCCM-10497)

A strain of *Streptomyces* sp. CJPV 975652 was used.

A culture method was as follows: 50 mℓ of a preculture medium (Table 6) was placed in an 500 mℓ Erlenmeyer flask and pressure-sterilized at 121°C for 20 minutes. A culture piece (0.5 cm x 0.5 cm) (containing the strain) that had been cultured in a plate medium for 2 weeks was inoculated on the sterilized preculture medium and cultured in a rotary stirring incubator at 250 rpm at 28°C for 2 days.

50 mℓ of a main medium (Table 7) that had been pressure-sterilized at 121°C for 20 minutes was placed in a 500 mℓ Erlenmeyer flask. 10% (5mℓ, v/v) of the resultant preculture was inoculated on the main medium and cultured in a rotary stirring incubator at 250 rpm at 28°C for 5 days. From 2 days after the culture, 0.5 g/L of sterilized compactin (in the form of sodium salt) was added twice a day to induce the conversion of compactin to pravastatin. After 10 mℓ of a fermentation broth was taken and centrifuged at 450xg for 10 minutes, PMV (Packed Mycelium Volume) was measured to evaluate the growth of the strain.

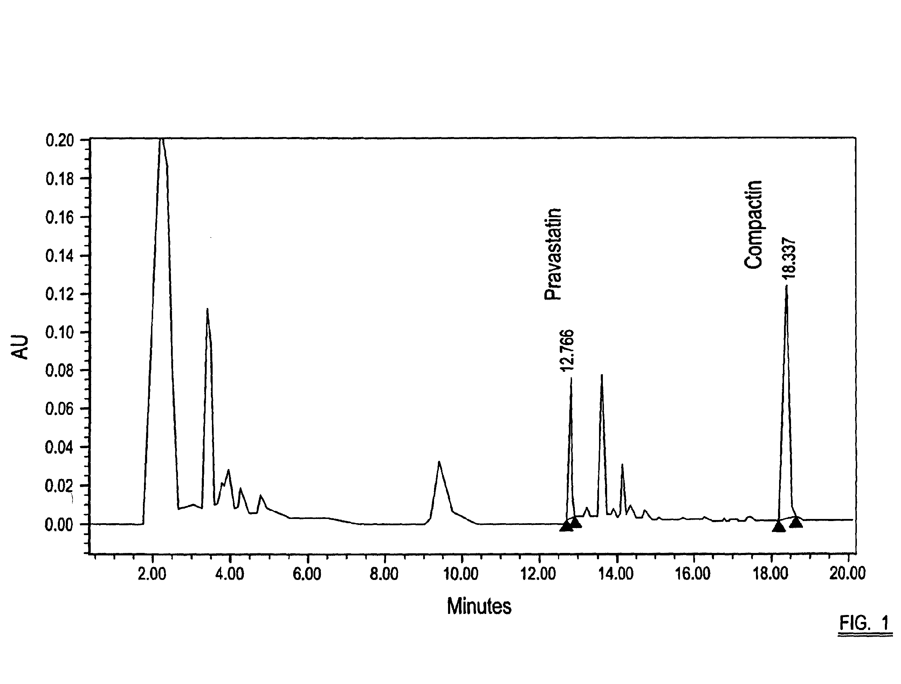

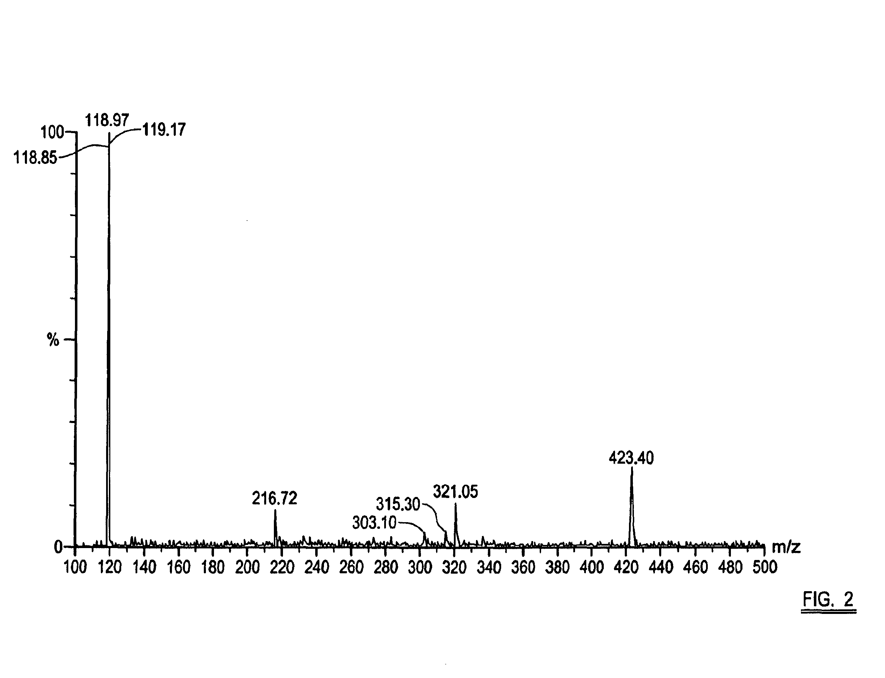

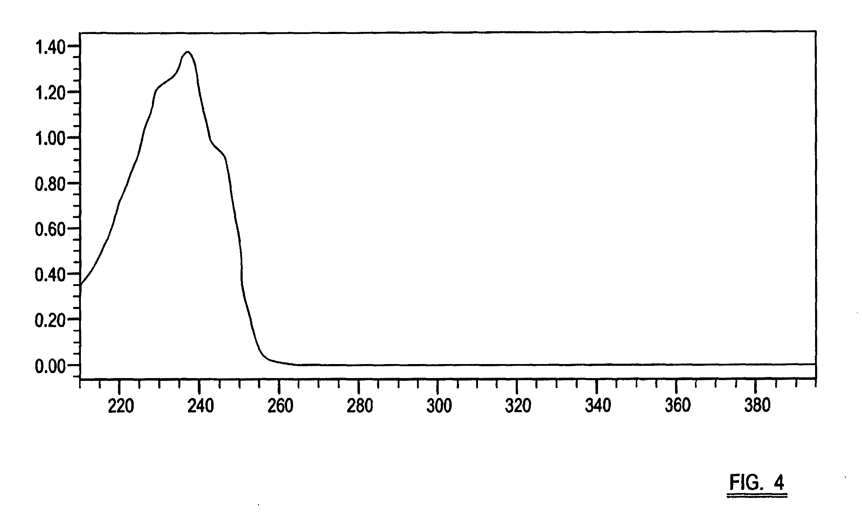

As a result, pH of the fermentation broth was 7.50 and PMV was 23%. HPLC was performed using waters C18 column (3.9 x 300 mm) and Shimadzu HPLC system (Model LC-10AD). For HPLC analysis, 400 *µ*ℓ of ethanol was added to 600 *µ*ℓ of the fermentation broth and centrifuged for 15-30 minutes. Here, a mixture of methanol: triethylamine: acetic acid: water (500 : 1 : 1: 500) was used as a mobile phase and the flow rate of the mobile phase was 1 ml/min. As a result of the HPLC analysis, pravastatin in the fermentation broth was identified (FIG. 1). LC-MS and UV spectra also revealed pravastatin (FIGS. 2, 3, and 4). Here, PDA2996 (Waters) was used for LC (liquid chromatography) and Ultima-PT (Micro-Mass) was used for MS (mass spectrometry). MS analysis was performed in negative mode under the conditions of 1,970 V of capillary tube voltage, 97 V of cone voltage, and 13 eV of collision energy.

In FIG. 1, the retention time of pravastatin was 12.766 minutes (area: 401298) and the retention time of compactin was 18.337 minutes (area: 230258). According to the HPLC chromatogram of FIG. 1, the ratio of the area of pravastatin to the sum of the areas of compactin and pravastatin was about 64%. This demonstrated that the conversion ratio of compactin to pravastatin was very high by about 64% or more.

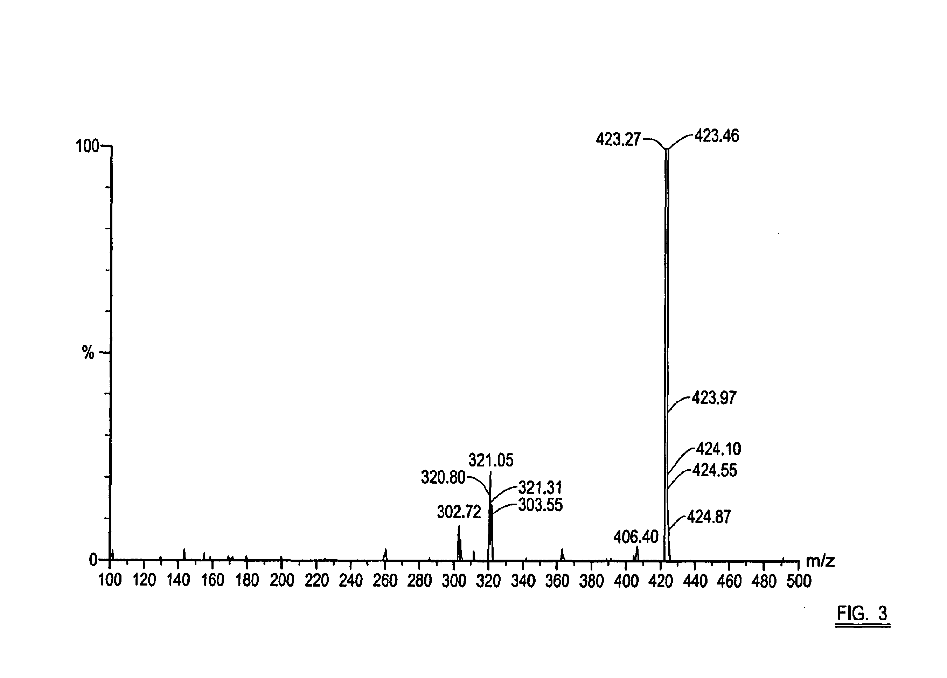

In FIGS. 2 and 3, a peak of m/z 423 indicates pravastatin. Since pravastatin was measured in negative mode, it is considered that the peak is for a sodium-free form of pravastatin. A peak of m/z 321 is a specific peak of pravastatin that indicates a daughter type substance obtained by fragmentation of pravastatin.

**Table 6:**

| Preculture medium of *Streptomyces* sp. CJPV 975652 | |
|---|---|
| Component | Content |
| Yeast extract | 0.1 % |
| Soybean powder | 1.5% |
| NaNO₃ | 0.5% |
| Glucose | 1.5% |
| Glycerol | 0.25% |
| CaCO₃ | 0.4% |

**Table 7:**

| Main culture medium of *Streptomyces* sp. CJPV 975652 | |
|---|---|
| Component | Content |
| Yeast extract | 0.1% |
| Soybean powder | 2.0% |
| Peptone | 0.5% |
| NaNO₃ | 0.5% |
| Glucose | 3.0% |
| Glycerol | 0.25% |
| CaCO₃ | 0.4% |
| NaCI | 0.5% |

### Example 3: Purification of pravastatin

After the culture was completed, 3 L of the microorganism culture was diluted with 2x distilled water and diatomite (3% volume) was added thereto with stirring. The resultant solution was filtered with a filter funnel packed with diatomite. Then, the filtrate was adsorbed on a column packed with 500ml of HP-20 resin, washed with a sufficient amount of water, and eluted with a 50% ethanol solution. The eluted solution was discolored using activated carbon and alumina resin and concentrated under a reduced pressure.

The crude concentrate was adsorbed on a column packed with 200 ml of XAD-1600 (Amberlite) resin and washed with 10% ethanol. Thereafter, pravastatin was separated with a 40% ethanol solution. The separated solution was concentrated under a reduced pressure, followed by crystallization with ethanol and ethylacetate, filtration, and drying under a reduced pressure, to give 1.3 g of pravastatin as a white crystal. Pravastatin thus obtained was subjected to H-NMR analysis. Here, Brucker ARX400 FT-NMR was used as a NMR machine and MeOH-d4 was used as a solvent.

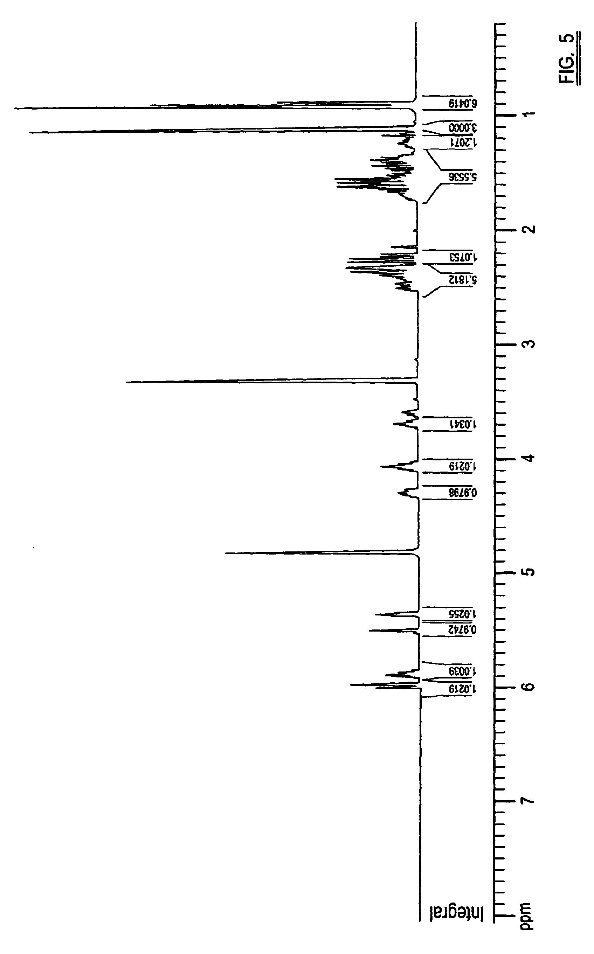

The H-NMR analysis result is shown in FIG. 5. The assignment of each chemical shift of FIG. 5 is presented in Table 8 below and the resultant compound is represented by the following Formula 7.

**Table 8:**

| Assignments of chemical shifts | | | |
|---|---|---|---|
| Chemical shift (ppm) | Assignment | Remark | Number of protons |
| 0.91 | 3"-CH3 | t | |
| 0.92 | 2'-CH3 | d | |
| 1.12 | 2"-CH3 | d | |
| 1.19 | H6b | m | |
| 1.36 | H7a | m | |
| 1.36 | H7b | m | |
| 1.46 | H3"b | m | |
| 1.53 | H4a | m | 10 |
| 1.54 | M6a | m | |
| 1.57 | H4b | m | |
| 1.58 | H7'a | m | |
| 1.61 | H3"a | m | |
| 1.67 | H1' | m | |
| 2.24 | H2a | dd | |
| 2.34 | H2b | dd | |
| 2.35 | H2" | m | |
| 2.36 | H8'a | m | |
| 2.41 | H2' | m | 4 |
| 2.47 | H7'b | m | |
| 3.69 | H5 | m | |
| 4.06 | H3 | m | |
| 4.29 | H6' | m | |
| 5.36 | H8' | brs | |
| 5.50 | H5' | brs | |
| 5.88 | H3' | dd | |
| 5.98 | H4' | dd | |

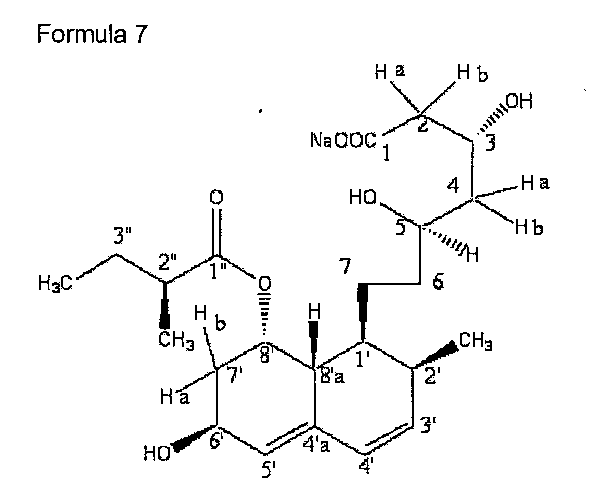

As shown in FIG. 5, pravastatin was identified.

According to *Streptomyces* sp. CJPV 975652 (KCCM-10497) of the present invention, compactin can be converted to pravastatin in high efficiency.

According to a method for producing provastatin using *Streptomyces* sp. CJPV 975652 (KCCM-10497) of the present invention, pravastatin can be produced in high yield.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A *Streptomyces* sp. CJPV 975652 (KCCM-10497) capable of converting compactin to pravastatin.

2. A method for producing pravastatin using a strain of *Streptomyces* sp. CJPV 975652 (KCCM-10497), which comprises:
culturing the strain of *Streptomyces* sp. CJPV 975652 (KCCM-10497) in a compactin-containing medium.

3. A method according to Claim 2, wherein the method further comprises recovering pravastatin from the culture.
